(19)

Europäisches
Patentamt
European
Patent Office
Office européen
des brevets

(11)     **EP 3 603 802 A1**

(12)     **EUROPEAN PATENT APPLICATION**

(43) Date of publication:
**05.02.2020 Bulletin 2020/06**

(51) Int Cl.:
**B01J 23/78** [(2006.01)]     **B01J 23/88** [(2006.01)]
**B01J 23/89** [(2006.01)]

(21) Application number: **19198504.3**

(22) Date of filing: **07.11.2008**

(84) Designated Contracting States:
**AT BE BG CH CY CZ DE DK EE ES FI FR GB GR HR HU IE IS IT LI LT LU LV MC MT NL NO PL PT RO SE SI SK TR**

(62) Document number(s) of the earlier application(s) in accordance with Art. 76 EPC:
**08877987.1 / 2 353 712**

(71) Applicant: **Clariant Catalysts (Japan) K.K. Tokyo 113-0021 (JP)**

(72) Inventors:
• **Mishima, Yuji**
**Toyama-shi, Toyama, 939-2753 (JP)**

• **Hirahara, Shinya**
**Toyama-shi, Toyama, 939-2753 (JP)**
• **Kondakari, Nobuaki**
**Toyama-shi. Toyama, 939-2753 (JP)**

(74) Representative: **Baronetzky, Klaus**
**Splanemann**
**Patentanwälte Partnerschaft**
**Rumfordstrasse 7**
**80469 München (DE)**

Remarks:
This application was filed on 20-09-2019 as a divisional application to the application mentioned under INID code 62.

(54)     **DEHYDROGENATION CATALYST FOR ALKYL AROMATIC COMPOUNDS EXHIBITING HIGH PERFORMANCE IN THE PRESENCE OF HIGH-CONCENTRATION CO2 AND DEHYDROGENATION PROCESS USING THE SAME**

(57)     The object of the present invention is to provide a catalyst for preparation of an alkenyl aromatic compound by dehydrogenating an alkyl aromatic compound, a process for preparation thereof, and a method for using it. Under the much carbon dioxide-containing dehydrogenation reaction condition in which $CO_2$ is present in a molar ratio of 0.015 to 0.20 based on an aromatic compound in a material gas, as the catalyst being capable of producing the alkenyl aromatic compound in the high yield, the catalyst which comprises an iron and an alkali metal, and comprises about 13 to about 60 % by weight of a rare earth element calculated as an oxide is contacted with a reaction gas.

EP 3 603 802 A1

**Description**

[Field of the Invention]

The present invention relates to a catalyst for preparing an alkenyl aromatic compound by dehydrogenating an alkyl aromatic compound, which is used in a reaction condition that $CO_2$ is present in a molar ratio of 0.015 to 0.20 based on an entire aromatic molecular in a material, a process for preparation thereof and a process for dehydrogenation using it.

[Description of the Related Art]

A styrene monomer which constitutes one of the alkyenyl aromatic compounds is an important industrial material which is widely used as a material such as polystyrene, an ABS resin or a synthetic rubber, and is industrially produced by reacting ethylbenzene under a dilute condition mainly using a steam by means of a catalyst having an iron oxide and an alkali metal, in particular potassium as main components in insulation type-dehydrogenation reactors which are doubly or triply arranged in series. But, the dehydrogenation reaction of ethylbenzen to the styrene monomer is an equilibrium reaction, and thus the reaction is largely prevented by the generated hydrogen. Also, because the dehydrogenation reaction is an endothermic reaction, a proceeding of the reaction and a decreasing of the reaction temperature are simultaneously taken place, resulting in a disadvantageous with regard to a reaction rate. For these reasons, it is difficult to obtain styrene in a high yield using a production equipment in which the above-mentioned dehydrogenation reactors are arranged in series.

Then, as described in the nonpatent reference 1, the patent reference 1 and the patent reference 2, processes are proposed and made practicable that after the hydrogen generated from the dehydrogenation reaction of ethylbenzen to the styrene monomer is selectively oxidized by means of oxygen, hydrogen is partially removed, and thus the equilibrium of the reaction sifts to the product system, as well as with a heat of the oxidation reaction compensates the decreasing of the temperature by the dehydrogenation reaction. However, in such processes, oxygen added in the oxidation step partially reacts with a hydrocarbon other than hydrogen to generate $CO_2$. The dehydrogenation catalyst disposed in the downstream oxidation step is generally used as the catalyst having the iron oxide and potassium as main components, and as described in the nonpatent reference 2 and the nonpatent reference 3, $CO_2$ results in a significant decreasing in a conversion and a selectiveness of the dehydrogenation catalyst. Therefore, this causes an industrial disadvantage.

In an improvement of the dehydrogenation catalyst of ethylbenzene, when a reaction in which a conventional preparation process, i.e. a preparation process which only comprises diluting ethylbenzene by means of a steam without the oxidation step is carried out under a mild condition that $CO_2$ is generated to a less extent, a variety of improved process are proposed, for example, a modification of the composition in catalyst as described in the patent reference 3 and the patent reference 4, as well as a variation in a pore size distribution of a material as described in the patent reference 5. However, it has not proposed that the dehydrogenation catalyst with a high yield is particularly used to the specific reaction condition, for example, the highly-concentrated $CO_2$ condition which is employed in the dehydrogenation step disposed in the downstream oxidation step as above-mentioned.

Nonpatent reference 1: "petrochemical process", (2001), p86, Ed. The Japan Petroleum Institute, published by Kodansha Ltd.

Nonpatent reference 2: Catalyst, 29, (8), 641, (1987)
Nonpatent reference 3: Petrotech, Vol. 29, No. 3, p158 (2006)

Patent reference 1: US 4565898
Patent reference 2: JP-A H05-38800
Patent reference 3: JP-A H03-11812
Patent reference 4: JP-A S64-45320
Patent reference 5: Japanese Patent No. 3881376

[Summary of the Invention]

Problems to be Resolved by the Invention

To solve the above-mentioned disadvantage in the related art, the present invention relates to a catalyst for preparing an alkenyl aromatic compound by dehydrogenating an alkyl aromatic compound, and therefore it is the object of the present invention to provide a high performance dehydrogenation catalyst of an alkyl aromatic compound in the presence of a highly concentrated $CO_2$, a process for preparation thereof and a process for dehydrogenation using it.

Means of Solving the Problems

We have diligently investigated and found that the catalyst which comprises about 13 to about 60 % by weight of the rare earth element calculated as the oxide weight in the alkali metal and iron compound-containing dehydrogenation catalyst has a very high yield of the alkenyl aromatic compound under the condition of the highly concentrated $CO_2$.

Comprising about 13 to about 60 % by weight of the rare earth element calculated as the oxide weight in the alkali metal and iron compound-containing dehydrogenation catalyst makes it possible to prepare the catalyst being capable of producing the alkenyl aromatic compound in high yield even under the reaction condition that the concentration of $CO_2$ is present in a molar ratio of 0.015 to 0.20 based on the aromatic compound in the material gas.

. In other words, the present invention relates to the dehydrogenation catalyst of an alkyl aromatic compound, which is used in a system which comprises at least a steam, $CO_2$ and the alkyl aromatic compound and in which $CO_2$ is present in a molar ratio of 0.015 to 0.20 based on an aromatic compound in a material gas, which comprises, based on a total weight of the catalyst, about 30 to about 86 % by weight of an iron compound calculated as $Fe_2O_3$, about 1 to about 50 % by weight of an alkali metal calculated as an alkali metal oxide and about 13 to about 60 % by weight of a rare earth element calculated as an oxide.

Furthermore, the dehydrogenation catalyst of an alkyl aromatic compound is used in a dehydrogenation step (2) for preparation steps of an alkenyl aromatic compound, comprising an oxidation step (1) for mixing the material gas comprising at least hydrogen, the steam and the alkyl aromatic compound with an oxygen-containing gas to oxidatively react a part of hydrogen in the presence of an oxidation catalyst, and the dehydrogenation step (2) for producing the alkenyl aromatic compound by means of the dehydrogenation catalyst of the alkyl aromatic compound in a reaction gas containing a by-product $CO_2$ eluting from the oxidation step, and wherein the dehydrogenation catalyst of the alkyl aromatic compound is used in a reaction condition that a concentration of $CO_2$ in the reaction gas is present in a molar ration of 0.015 to 0.20 based on a total flow rate of all aromatic molecules consisting of an unreacted alkyl aromatic compound contained in the material gas and the alkenyl aromatic compound in a product.

Furthermore, the alkyl aromatic compound is ethylbenzene, and the alkenyl aromatic compound is styrene monomer.

Furthermore, the iron compound is iron oxide.

Furthermore, the iron compound is an oxide comprising potassium, and its composition is represented by a chemical formula $K_2O \cdot (Fe_2O_3)_n$ in which n is an integral number of 1 to 11.

Furthermore, the alkali metal is a sodium compound or a potassium compound.

Furthermore, the alkali metal is potassium carbonate:

Furthermore, the rare earth element is cerium or lanthanum.

Also, the dehydrogenation catalyst of an alkyl aromatic compound is used in a system which comprises at least a steam, $CO_2$ and the alkyl aromatic compound and in which $CO_2$ is present in a molar ratio of 0.015 to 0.20 based on an aromatic compound in a material gas, which comprises, based on a total weight of the catalyst, about 30 to about 85.5 % by weight of an iron compound calculated as $Fe_2O_3$, about 13 to about 60 % by weight of cerium or lanthanum calculated as an oxide weight, about 1 to about 50 % by weight of an alkali metal calculated as an alkali metal oxide, about 0.2 to about 10 % by weight of an alkali earth metal calculated as an alkali earth metal oxide and about 0.2 to about 10 % by weight of a molybdenum or tungsten compound calculated as $MoO_3$ or $WO_3$.

Also, the dehydrogenation catalyst of an alkyl aromatic compound is used in a system which comprises at least a steam, $CO_2$ and the alkyl aromatic compound and in which $CO_2$ is present in a molar ratio of 0.015 to 0.20 based on an aromatic compound in a material gas, which comprises, based on a total weight of the catalyst, about 30 to about 85.5 % by weight of an iron compound calculated as $Fe_2O_3$, about 13 to about 60 % by weight of cerium or lanthanum calculated as an oxide weight, about 1 to about 50 % by weight of an alkali metal calculated as an alkali metal oxide, about 0.2 to about 10 % by weight of an alkali earth metal calculated as an alkali earth metal oxide, about 0.2 to about 10 % by weight of a molybdenum or tungsten compound calculated as $MoO_3$ or $WO_3$ and about 1 to 200 ppm of a noble metal.

Furthermore, the noble metal is selected from the group consisting of palladium, platinum, iridium, rhodium and ruthenium.

A process for preparation of a sintered dehydrogenation catalyst of an alkyl aromatic compound, which comprises the steps of: mixing the dehydrogenation catalyst of the alkyl aromatic compound with sufficient water to prepare an extrudable mixture, to prepare to the extrudable mixture; molding the extrudable mixture to make a pellet; after drying the pellet, sintering it to make the finished catalyst.

Furthermore, a process for dehydrogenation of an alkyl aromatic compound, which comprises contacting the dehydrogenation catalyst of the alkyl aromatic compound with a reaction gas which comprises at least a steam, $CO_2$ and the alkyl aromatic compound and in which $CO_2$ is present in a molar ratio of 0.015 to 0.20 based on an aromatic compound in a material gas, to produce an alkenyl aromatic compound.

Effect of the Invention

According to the present invention, the catalyst which comprises about 13 to about 60 % by weight of the rare earth element calculated as the oxide weight in the alkali metal and iron compound-containing dehydrogenation catalyst is used, and thus makes it possible to prepare the dehydrogenation catalyst which can be used in industrial scale and by which the decreasing of the yield due to carbon dioxide is largely improved, under the dehydrogenation condition that much carbon dioxide could not be conventionally contained.

[Best Mode for Practicing the Invention]

The present invention is explained in details below.

In the dehydrogenation reaction of the alkyl aromatic compound diluted with the steam to the alkenyl aromatic compound, the dehydrogenation reaction condition in which much $CO_2$ is contained means that $CO_2$ in the system is present in a molar ration of 0.015 to 0.20 based on a total flow rate of all aromatic molecules such as an unreacted alkyl aromatic compound and the alkenyl aromatic compound in a product. $CO_2$ contained in the reaction system is produced by the secondary reaction in the step for selectively oxidizing hydrogen generated from the dehydrogenation reaction with the oxidation catalyst.

More particular, in a process consisting of the following steps: the oxidation step for mixing the material gas consisting of a mixture of the unreacted alkyl aromatic compound, the alkenyl aromatic compound, hydrogen and the steam with an oxygen-containing gas to oxidatively react a part of hydrogen in the presence of an oxidation catalyst, and the dehydrogenation step for dehydrogenating a reaction gas containing a by-product $CO_2$ by reacting the unreacted alkyl aromatic compound, the alkenyl aromatic compound, hydrogen, the steam and a part of the alkyl aromatic compound eluting from the oxidation step with oxygen by means of the dehydrogenation catalyst to produce the alkenyl aromatic compound, the dehydrogenation catalyst is used in the dehydrogenation step in which the concentration of $CO_2$ disposed in the downstream oxidation step is enhanced. The concentration of $CO_2$ in the reaction gas in the downstream oxidation step is in a molar ration of 0.015 to 0.20 based on a total flow rate of all aromatic molecules contained in the material gas comprising an unreacted alkyl aromatic compound and the alkenyl aromatic compound. In addition, the oxidation step is well-known, and the product stream consisting of the unreacted hydrocarbon, the dehydrogenated hydrocarbon, the steam and hydrogen after of a conventional dehydrogenation step is subjected to the selective oxidation in the contacting the oxygen-containing gas with an oxidized catalytic bed. A catalyst used in the dehydrogenation step and the oxidation step prior to the reaction process which utilizes the catalyst according to the invention is well-known, and is not particularly limited. For example, the dehydrogenation catalyst used in the prior process is consisted of an alkali metal-promoting iron compound, and the oxidation catalyst is any one of catalysts which selectively oxidize hydrogen and is consisted of the noble metal in the VIII group of the periodic table, such as platinum, on an ?-alumina.

The dehydrogenation catalyst of the alkyl aromatic compound according to the present invention is useful as the dehydrogenation catalyst by means of which the alkenyl aromatic compound is generated by contacting the alkyl aromatic compound with the steam, in particular, is useful for promoting the dehydrogenation of ethylbenzene if contacting the ethylbenzene with the steam is carried out to produce styrene, and is adequately useful for the dehydrogenation reaction step in which the concentration of $CO_2$ is enhanced after the oxidation step. The process for dehydrogenation using the catalyst according to the invention is carried out as a successive operation utilizing a fixed bed, and the fixed bed may be consist of one stage or be a series of stages which are consist of same or different catalysts in one reactor or two more reactors. Then, other types of reactors and the reactor form also can be used for the dehydrogenation process.

In the process for dehydrogenation using the catalyst according to the present invention, a mixing ratio of the steam to a total flow rate of the alkyl aromatic compound and the alkenyl aromatic compound contained in the material gas after the oxidation step is particularly not limited, however, is usually from 3 to 15 of the molar ratio, preferably 5 to 13. The reaction temperature of a catalyst layer in which the gaseous material with the composition is contacted with the catalyst layer is usually 500 °C or more, preferably 530 °C or more. Also, the reaction pressure may be 0.1 to 3 atm in absolute. Preferably, the supply rate of the alkyl aromatic compound is 0.1 to 10 $h^{-1}$ in Liquid Hourly Space Velocity (LHSV).

In the catalyst according to the present invention which is used for the dehydrogenation step of the alkyl aromatic compound in the reaction system in which much $CO_2$ is contained, it is important to comprise about 13 to about 60 % by weight of the rare earth element as the oxide weight. More the content of the rare earth element, higher the conversion of the alkyl aromatic compound. Also, if the content of the rare earth element is less than 13 % by weight, the industrially useful conversion cannot be obtained, and on the other hand, if it is more than 60 % by weight, the conversion is higher, but it is not practical due to an expensive material of the catalyst. Preferably, the rare earth element for the catalyst is, in particular, cerium or lanthanum both of which are basic.

Also, in the composition of the catalyst according to the present invention, the composition other than the rare earth element as mentioned above may be a general composition in which the catalyst comprises the iron oxide and the alkali metal.

The iron material for the present invention is basically red, yellow, brown and black iron oxides with a variety form. It is added as the red iron oxide $Fe_2O_3$ or the yellow iron oxide $Fe_2O_3 \cdot H_2O$, but it is not necessarily so, and an iron oxide precursor, i.e. goethite or a basic sodium carbonate which can convert to the iron oxide during sintering the catalyst can be used. As the iron compound, ferrite can also be used. Preferably, potassium ferrite can be used.

The alkali metal is selected from the group consisting of lithium, sodium, potassium, rubidium and cesium. Also, in addition to the iron oxide-alkali metal, other elements may be comprised in an amount that it has already proposed as a promoter. For example, to improve the selectiveness, the alkali earth metal containing molybdenum or tungsten, or barium, francium and radium, or the noble metal selected from the group consisting of palladium, platinum, iridium, rhodium and ruthenium may be contained.

In one embodiment of the composition of the catalyst according to the present invention, it is possible to comprise about 30 to about 86 % by weight of the iron compound calculated as $Fe_2O_3$, about 1 to about 50 % by weight of an alkali metal calculated as an alkali metal oxide and about 13 to about 60 % by weight of a rare earth element calculated as an oxide.

Moreover, a suitable embodiment of the composition of the catalyst according to the present invention is listed as follows:

| | |
|---|---|
| $Fe_2O_3$ | 30 to 85.5 % by weight |
| Alkali metal (in the oxide weight) | 1 to 50 % by weight |
| Alkali earth metal (in the oxide weight) | 0.2 to 10 % by weight |
| $MoO_3$ or $WO_3$ | 0.2 to 10 % by weight |
| Rare earth element (in the oxide weight) | 13 to 60 % by weight. |
| Noble metal | 1 to 200 ppm |

The catalyst according to the present invention is finally sintered in the range 500 to 1100 °C, and thus any compounds can be used, if the compounds which can be used as the promoter component can be decomposed by thermally treating them. Usually, in consideration of an ease of access and an economic efficiency, it is preferred to use a carbonate or a hydroxide as the material of alkali the metal, the alkali earth metal or the rare earth element. Preference is given to use p-ammonium molybdate and p-ammonium tungstate or molybdenum oxide or tungsten oxide as molybdenum or tungsten.

The catalyst according to the present invention can be prepared by a usual method. It is prepared by mixing an essential component as mentioned above with an optional component, and sintering the generated mixture. Preferably, the material for the catalyst is moistly kneaded. In this case, it is necessary for an amount of the added water in the kneading to adapt to the amount in the subsequent extruding. Its amount, depending on the kind of the material used, is usually 2 to 50 % by weight, and after sufficiently kneading it, it is extruded and then sintered to obtain a certain dehydrogenation catalyst. In the drying, it is an enough to remove free water possessed in the extrusion, and it is carried out at the temperature of 60 to 200 °C; preferably 70 to 180 °C. On the other hand, the sintering is carried out to thermally decompose each catalyst precursors possessed in a dry substance, to improve the physical stability of the catalyst pellet and to improve its performance, and is carried out at the temperature of 500 to 1100°C, preferably 500 to 900°C.

If the catalyst according to the present invention can be used as an usual fixed-bed catalyst, it has any shapes, for example, round, cylindrical, macaroni and bulk.

The present invention was explained in details based on the Examples and Comparative Examples as mentioned below. But, the invention is not limited to these Examples and Comparative Examples. Unless otherwise stated, part and % is based on the weight.

[Example]

[Example 1]

527.3 g of red iron oxide (hematite crystal structure), 331.6 g of potassium carbonate and 376.6 g of cerium carbonate hydrate were weighed into a kneading machine and deionized water was added dropwise with stirring to form a paste. The mixed paste obtained was sintered at 900 °C for 2 hours, and milled, and then glanulated by means of a sieve having an opening size of 2.36 mm and a sieve having an opening size of 1.40 mm. This gave a dehydrogenation catalyst comprising 56.0 % of iron oxide calculated as $Fe_2O_3$, 24.0% of potassium calculated as $K_2O$ and 20.0 % of cerium calculated as $CeO_2$. A reaction test was carried out with using the dehydrogenation catalyst obtained under following reaction condition.

amount of catalyst 20cc

Liquid Hourly Space Velocity (LHSV) of material ethylbenzene = 5.0 h$^{-1}$

(diluted $H_2O$ flow rate) ÷ (material ethylbenzene flow rate) = 1.60 wt/wt (ratio by weight)

reaction pressure = 61 kPa (absolute pressure)

reaction temperature = 600 °C

After confirming that a conversion of the reactant calculated by the following equation had been stable, an addition of $CO_2$ gas into the above-described reaction condition was started at a flow rate corresponding to ($CO_2$ flow rate) ÷ (material ethylbenzene flow rate) = 0.03 after 72 hours from the start of flowing of ethylbenzene. The conversion was measured after 23 hours from the start of flowing of $CO_2$.

Conversion (mole%) = (supplied ethylbenzene (mole) - discharged ethylbenzene (mole)) ÷ supplied ethylbenzene (mole)× 100

Then, the flow rate of the added $CO_2$ was increased to a flow rate corresponding to ($CO_2$ flow rate) ÷ (material ethylbenzene flow rate) = 0.06 after 24 hours from the start of flowing of $CO_2$. The conversion was measured after 47 hours from the start of flowing of $CO_2$. In addition, the flow rate of the added $CO_2$ was increased to a flow rate corresponding to ($CO_2$ flow rate) ÷ (material ethylbenzene flow rate) = 0.13 after 48 hours from the start of flowing of $CO_2$. The conversion was measured after 71 hours from the start of flowing of $CO_2$. The following table shows the conversions at the each concentration under flowing of $CO_2$.

[Comparative Example 1]

Ethylbenzene conversions at the each concentration under flowing of $CO_2$ were measured in the same manner as in Example 1 except for preparing a catalyst comprising 76.0 % of iron oxide calculated as $Fe_2O_3$ and 24.0 % of potassium calculated as $K_2O$ by using715.6 g of red iron oxide (hematite crystal structure) and 331.6 g of potassium carbonate. The obtained results are shown in Table 1.

[Example 2]

697.3 g of red iron oxide (hematite crystal structure), 166.5 g of potassium carbonate and 405.4 g of cerium carbonate hydrate were weighed into a kneading machine and deionized water was added dropwise with stirring to form a paste. The mixed paste obtained was sintered at 900 °C for 2 hours, and milled, and then glanulated by means of a sieve having an opening size of 2.36 mm and a sieve having an opening size of 1.40 mm. This gave a dehydrogenation catalyst comprising 68.8 % of iron oxide calculated as $Fe_2O_3$, 11.2 % of potassium calculated as $K_2O$ and 20.0 % of cerium calculated as $CeO_2$. A reaction test was carried out with using the dehydrogenation catalyst obtained under following reaction condition.
amount of catalyst 20cc

Liquid Hourly Space Velocity (LHSV) of material ethylbenzene = 5.0 $h^{-1}$

(diluted $H_2O$ flow rate) ÷ (material ethylbenzene flow rate) = 1.60 wt/wt (ratio by weight)

reaction pressure = 61 kPa (absolute pressure)

reaction temperature = 600 °C

After confirming that a conversion had been stable, an addition of $CO_2$ gas into the above-described reaction condition was started at a flow rate corresponding to ($CO_2$ flow rate) ÷ (material ethylbenzene flow rate) = 0.03 after 72 hours from the start of flowing of ethylbenzene. The conversion was measured after 23 hours from the start of flowing of $CO_2$. Table 2 shows conversions at the each concentration under flowing of $CO_2$.

[Example 3].

Conversions at the each concentration under flowing of $CO_2$ were measured in the same manner as in Example 2 except for preparing a catalyst comprising 68.8 % of iron oxide calculated as $Fe_2O_3$, 11.2 % of potassium calculated as $K_2O$, 10.0 % of cerium calculated as $CeO_2$ and 10.0 % of lanthanum calculated as $La_2O_3$ by using 697.3 g of red iron oxide (hematite crystal structure), 166.5 g of potassium carbonate, 202.7 g of cerium carbonate hydrate and 187.3 g of lanthanum carbonate octahydrate. The obtained results are shown in Table 2.

[Comparative Example 2]

Conversions at the each concentration under flowing of $CO_2$ were measured in the same manner as in Example 2 except for preparing a catalyst comprising 88.8 % of iron oxide calculated as $Fe_2O_3$ and 11.2 % of potassium calculated as $K_2O$ by using 900.0 g of red iron oxide (hematite crystal structure) and 166.5 g of potassium carbonate. The obtained results are shown in Table 2.

[Example 4]

Conversions at the each concentration under flowing of $CO_2$ were measured in the same manner as in Example 2 except for preparing a catalyst comprising 48.8 % of iron oxide calculated as $Fe_2O_3$, 11.2 % of potassium calculated as $K_2O$ and 40.0 % of cerium calculated as $CeO_2$ by using 494.6 g of red iron oxide (hematite crystal structure), 166.5 g of potassium carbonate and 810.8 g of cerium carbonate hydrate. The obtained results are shown in Table 2.

[Example 5]

Conversions at the each concentration under flowing of $CO_2$ were measured in the same manner as in Example 2 except for preparing a catalyst comprising 75.8 % of iron oxide calculated as $Fe_2O_3$, 11.2 % of potassium calculated as $K_2O$ and 13.0 % of cerium calculated as $CeO_2$ by using 768.0 g of red iron oxide (hematite crystal structure), 166.5 g of potassium carbonate and 263.4 g of cerium carbonate hydrate. The obtained results are shown in Table 2.

[Comparative Example 3]

Conversions at the each concentration under flowing of $CO_2$ were measured in the same manner as in Example 2 except for preparing a catalyst comprising 83.8 % of iron oxide calculated as $Fe_2O_3$, 11.2 % of potassium calculated as $K_2O$ and 5.0 % of cerium calculated as $CeO_2$ by using 849.1 g of red iron oxide (hematite crystal structure), 166.5 g of potassium carbonate and 101.3 g of cerium carbonate hydrate. The obtained results are shown in Table 2.

[Example 6]

Conversions at the each concentration under flowing of $CO_2$ were measured in the same manner as in Example 2 except for preparing a catalyst comprising 67.8 % of iron oxide calculated as $Fe_2O_3$, 11.2 % of potassium calculated as $K_2O$, 20.0 % of cerium calculated as $CeO_2$, 0.5 % of calcium calculated as CaO and 0.5 % of molybdenum calculated as $MoO_3$ by using 687.1 g of red iron oxide (hematite crystal structure), 166.5 g of potassium carbonate, 405.4 g of cerium carbonate hydrate, 6.7 g of calcium hydroxide and 5.1 g of molybdenum trioxide. The obtained results are shown in Table 3.

[Comparative Example 4]

Conversions at the each concentration under flowing of $CO_2$ were measured in the same manner as in Example 2 except for preparing a catalyst comprising 87.8 % of iron oxide calculated as $Fe_2O_3$, 11.2 % of potassium calculated as $K_2O$, 0.5 % of calcium calculated as CaO and 0.5 % of molybdenum calculated as $MoO_3$ by using 889.9 g of red iron oxide (hematite crystal structure), 166.5 g of potassium carbonate, 6.7 g of calcium hydroxide and 5.1 g of molybdenum trioxide. The obtained results are shown in Table 3.

[Example 7]

Conversions at the each concentration under flowing of $CO_2$ were measured in the same manner as in Example 2 except for preparing a catalyst comprising 67.8 % of iron oxide calculated as $Fe_2O_3$, 11.2 % of potassium calculated as $K_2O$, 20.0 % of cerium calculated as $CeO_2$, 0.5 % of calcium calculated as CaO, 0.5 % of molybdenum calculated as $MoO_3$ and 100 ppm of palladium metal by using 687.1 g of red iron oxide (hematite crystal structure), 166.5 g of potassium carbonate, 405.4 g of cerium carbonate hydrate, 6.7 g of calcium hydroxide, 5.1 g of molybdenum trioxide and palladium nitrate solution. The obtained results are shown in Table 3.

[Table 1]

| Example | Composition | | | $CO_2 \div$ ethylbenzene ratio (mole / mole) | Ethylbenzene conversion (mole %) |
|---|---|---|---|---|---|
| | $Fe_2O_3$ | $K_2O$ | $CeO_2$ | | |
| Example 1 | 56.0 % | 24.0 % | 20.0 % | 0.03 | 38.3 |
| | | | | 0.06 | 28.2 |
| | | | | 0.13 | 18.6 |
| Comparative Example 1 | 76.0 % | 24.0 % | | 0.03 | 10.1 |
| | | | | 0.06 | 6.7 |
| | | | | 0.13 | 4.8 |

[Table 2]

| Example | Composition | | | | Ethylbenzene conversion (mole %) |
|---|---|---|---|---|---|
| | $Fe_2O_3$ | $K_2O$ | $CeO_2$ | $La_2O_3$ | |
| Example 2 | 68.8 % | 11.2 % | 20.0 % | | 53.5 |
| Example 3 | 68.8 % | 11.2 % | 10.0 % | 10.0 % | 36.6 |
| Comparative Example 2 | 88.8 % | 11.2 % | | | 26.5 |
| Example 4 | 48.8 % | 11.2 % | 40.0 % | | 68.1 |
| Example 5 | 75.8 % | 11.2 % | 13.0 % | | 49.8 |
| Comparative Example 3 | 83.8% | 11.2 % | 5.0 % | | 35.5 |

[Table 3]

| Example | Composition | | | | | | Ethylbenzene conversion (mole %) |
|---|---|---|---|---|---|---|---|
| | $Fe_2O_3$ | $K_2O$ | $CeO_2$ | CaO | $MoO_3$ | Pd | |
| Example 6 | 67.8 % | 11.2 % | 20.0 % | 0.5 % | 0.5 % | | 41.2 |
| Comparative Example 4 | 87.8 % | 11.2 % | | 0.5 % | 0.5 % | | 12.0 |
| Example 7 | 67.8 % | 11.2 % | 20.0 % | 0.5% | 0.5% | 100 ppm | 48.2 |

The above-mentioned Examples and Comparative Examples show that the catalyst according to the present invention can achieve a significantly high yield under a concentration high of carbon dioxide.

[Industrial Applicability]

The dehydrogenation catalyst of the present invention can produce the alkenyl aromatic compound in high yield under high carbon dioxide concentration in which the concentration of $CO_2$ is present in a molar ratio of 0.015 to 0.20 based on all aromatic molecules including the alkyl aromatics in the material gas without reduction of yield owing to $CO_2$ in a conventional method. Therefore, the dehydrogenation catalyst of the present invention, the process for preparing it and the dehydrogenating process using it contribute greatly to an optimum production system of the alkenyl aromatics.

**Claims**

1.   An dehydrogenation catalyst for producing an alkenyl aromatic compound from a gas comprising at least CO2 and an alkyl aromatic compound, the dehydrogenation catalyst comprises:

    30 to 86 wt % of an iron compound calculated as an iron oxide,
    1 to 50 wt % of an alkali metal calculated as an alkali metal oxide, and
    13 to 60 wt % of a rare earth element calculated as a rare earth oxide, wherein the wt % is based on the total weight of the dehydrogenation catalyst.

2.   The dehydrogenation catalyst of claim 1, wherein the alkyl aromatic compound is ethylbenzene, and the alkenyl aromatic compound is styrene monomer.

3.   The dehydrogenation catalyst of claim 1, wherein said alkali metal is selected from the group consisting of sodium, potassium and a mixture thereof.

4.   The dehydrogenation catalyst of claim 1, wherein said rare earth element is selected from the group consisting of cerium, lanthanum and a mixture thereof.

5.   The dehydrogenation catalyst of claim 1, wherein the dehydrogenation catalyst further comprises:

    0.2 to 10 wt % of an alkali earth metal calculated as an alkali earth metal oxide, and
    0.2 to 10 wt % of a transition metal calculated as a transition metal oxide,
    wherein the wt % is based on the total weight of the dehydrogenation catalyst.

6.   The dehydrogenation catalyst of claim 5, wherein said transition metal is selected from the group consisting of molybdenum, tungsten and a mixture thereof.

7.   The dehydrogenation catalyst of claim 1, wherein the dehydrogenation catalyst further comprises 1 to 200 ppm of a noble metal selected from the group consisting of palladium, platinum, iridium, rhodium, and ruthenium.

8.   A method for dehydrogenation of an alkyl aromatic compound, comprising:

    (1) oxidation step of mixing a material gas comprising hydrogen, steam, and an alkyl aromatic compound, optionally an alkenyl aromatic compound and an oxygen-containing gas comprising oxygen to form a reaction gas, wherein carbon dioxide is produced in the reaction gas, wherein said carbon dioxide is 0.015 to 0.20 molar ratio based on the total mole of the aromatic compounds in the material gas,

(2) dehydrogenation step of contacting the reaction gas with a dehydrogenation catalyst to produce an alkenyl aromatic compound, wherein the dehydrogenation catalyst comprises

30 to 86 wt % of an iron compound calculated as an iron oxide,

1 to 50 wt % of an alkali metal calculated as an alkali metal oxide, and

13 to 60 wt % of a rare earth element calculated as a rare earth oxide,

wherein the wt % is based on the total weight of the dehydrogenation catalyst.

9. The method for dehydrogenation of an alkyl aromatic compound of claim 8, wherein the alkyl aromatic compound is ethylbenzene, and the alkenyl aromatic compound is styrene monomer.

10. The method for dehydrogenation of an alkyl aromatic compound of claim 8, wherein said alkali metal is selected from the group consisting of sodium, potassium and a mixture thereof.

11. The method for dehydrogenation of an alkyl aromatic compound of claim 8, wherein said rare earth element is selected from the group consisting of cerium, lanthanum and a mixture thereof.

12. The method for dehydrogenation of an alkyl aromatic compound of claim 8, wherein the dehydrogenation catalyst further comprises:

0.2 to 10 wt % of an alkali earth metal calculated as an alkali earth metal oxide, and

0.2 to 10 wt % of a transition metal calculated as a transition metal oxide,

wherein the wt % is based on the total weight of the dehydrogenation catalyst.

13. The method for dehydrogenation of an alkyl aromatic compound of claim 12, wherein said transition metal is selected from the group consisting of molybdenum, tungsten and a mixture thereof.

14. The method for dehydrogenation of an alkyl aromatic compound of claim 12, wherein the dehydrogenation catalyst further comprises 1 to 200 ppm of a noble metal selected from the group consisting of palladium, platinum, iridium, rhodium, and ruthenium.

Europäisches
Patentamt

European
Patent Office

Office européen
des brevets

# EUROPEAN SEARCH REPORT

Application Number

EP 19 19 8504

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2008/090974 A1 (SUED CHEMIE CATALYSTS JAPAN IN [JP]; MISHIMA YUJI [JP]) 31 July 2008 (2008-07-31) * the whole document * & EP 2 106 852 A1 (SUED CHEMIE CATALYSTS JAPAN IN [JP]; MISHIMA YUJI [JP] SUED CHEMIE CAT) 7 October 2009 (2009-10-07) * abstract * * paragraphs [0017], [0029], [0030] * * example 1 * * claims 1,6-8 * | 1-6,8-13 | INV. B01J23/78 B01J23/88 B01J23/89 |
| X | US 4 460 706 A (IMANARI MAKOTO [JP] ET AL) 17 July 1984 (1984-07-17) * abstract * * column 1, line 49 - column 2, line 2 * * column 3, lines 13-24 * * example 1 * | 1-4,8-11 | |
| X | WO 99/49968 A1 (UNITED CATALYSTS INC [US]) 7 October 1999 (1999-10-07) * abstract * * page 8, line 14 - page 9, line 11 * * page 11, lines 1-19 * * page 17, lines 9-18 * * page 20, lines 12-15 * | 1-14 | TECHNICAL FIELDS SEARCHED (IPC) B01J C07C |
| X | US 2006/106267 A1 (KOWALESKI RUTH M [US] KOWALESKI RUTH MARY [US]) 18 May 2006 (2006-05-18) * abstract * * paragraphs [0036] - [0046], [0050], [0054] - [0056] * * examples 1,2 * | 1-6,8-13 | |

-/--

The present search report has been drawn up for all claims

| Place of search | Date of completion of the search | Examiner |
|---|---|---|
| Munich | 11 December 2019 | Fischbach, Malaika |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

Application Number

EP 19 19 8504

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2002/183573 A1 (COCCO RAYMOND A [US] ET AL) 5 December 2002 (2002-12-05)<br>* abstract *<br>* paragraphs [0002], [0009], [0011], [0012], [0022], [0026] - [0028] *<br>* example 9 *<br>* claims 1,3,8-10 *<br>----- | 1-4,8-11 | |
| | | | **TECHNICAL FIELDS SEARCHED (IPC)** |

The present search report has been drawn up for all claims

| Place of search | Date of completion of the search | Examiner |
|---|---|---|
| Munich | 11 December 2019 | Fischbach, Malaika |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
..............................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 19 19 8504

This annex lists the patent family members relating to the patent documents cited in the above-mentioned European search report.
The members are as contained in the European Patent Office EDP file on
The European Patent Office is in no way liable for these particulars which are merely given for the purpose of information.

11-12-2019

| Patent document cited in search report | | Publication date | Patent family member(s) | | Publication date |
|---|---|---|---|---|---|
| WO 2008090974 | A1 | 31-07-2008 | CA | 2676383 A1 | 31-07-2008 |
| | | | CN | 101631612 A | 20-01-2010 |
| | | | EP | 2106852 A1 | 07-10-2009 |
| | | | JP | 5096751 B2 | 12-12-2012 |
| | | | JP | 2008183492 A | 14-08-2008 |
| | | | KR | 20100014807 A | 11-02-2010 |
| | | | US | 2010087694 A1 | 08-04-2010 |
| | | | US | 2013053608 A1 | 28-02-2013 |
| | | | WO | 2008090974 A1 | 31-07-2008 |
| US 4460706 | A | 17-07-1984 | JP | H0311812 B2 | 18-02-1991 |
| | | | JP | S58177148 A | 17-10-1983 |
| | | | US | 4460706 A | 17-07-1984 |
| WO 9949968 | A1 | 07-10-1999 | AU | 3205499 A | 18-10-1999 |
| | | | CA | 2326538 A1 | 07-10-1999 |
| | | | CN | 1298323 A | 06-06-2001 |
| | | | CZ | 20003590 A3 | 15-08-2001 |
| | | | CZ | 20004346 A3 | 11-07-2001 |
| | | | EP | 1071508 A1 | 31-01-2001 |
| | | | ID | 28231 A | 10-05-2001 |
| | | | JP | 4136312 B2 | 20-08-2008 |
| | | | JP | 2002509790 A | 02-04-2002 |
| | | | PL | 343154 A1 | 30-07-2001 |
| | | | TW | 426546 B | 21-03-2001 |
| | | | WO | 9949968 A1 | 07-10-1999 |
| US 2006106267 | A1 | 18-05-2006 | AR | 051961 A1 | 21-02-2007 |
| | | | AU | 2005307751 A1 | 26-05-2006 |
| | | | BR | PI0518290 A2 | 11-11-2008 |
| | | | CA | 2587476 A1 | 26-05-2006 |
| | | | CN | 101115698 A | 30-01-2008 |
| | | | EP | 1814835 A1 | 08-08-2007 |
| | | | JP | 2008520685 A | 19-06-2008 |
| | | | KR | 20070086289 A | 27-08-2007 |
| | | | RU | 2385313 C2 | 27-03-2010 |
| | | | TW | 200631934 A | 16-09-2006 |
| | | | US | 2006106267 A1 | 18-05-2006 |
| | | | WO | 2006055651 A1 | 26-05-2006 |
| US 2002183573 | A1 | 05-12-2002 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4565898 A
- JP H0538800 A
- JP H0311812 A
- JP S6445320 A
- JP 3881376 B

**Non-patent literature cited in the description**

- petrochemical process. Kodansha Ltd, 2001, 86
- *Catalyst,* 1987, vol. 29 (8), 641
- *Petrotech,* 2006, vol. 29 (3), 158